(19) Europäisches Patentamt
European Patent Office
Office européen des brevets

(11) Veröffentlichungsnummer: **0 116 885**
**B1**

(12) # EUROPÄISCHE PATENTSCHRIFT

(45) Veröffentlichungstag der Patentschrift: **07.01.87**

(51) Int. Cl.⁴: **C 07 C 79/12**, C 07 C 76/02

(21) Anmeldenummer: **84101133.1**

(22) Anmeldetag: **04.02.84**

(54) **Verfahren zur Herstellung von fluorierten Nitroalkanen.**

(30) Priorität: **16.02.83 DE 3305202**

(43) Veröffentlichungstag der Anmeldung:
**29.08.84 Patentblatt 84/35**

(45) Bekanntmachung des Hinweises auf die Patenterteilung:
**07.01.87 Patentblatt 87/2**

(84) Benannte Vertragsstaaten:
**CH DE FR GB IT LI**

(56) Entgegenhaltungen:
**FR - A - 892 442**
**US - A - 2 864 853**
**US - A - 3 118 004**

**DOKLADY AKADEMII NAUK SSSR, Band 149, 1963, Moskau, A.I. TITOV "Der Ionenmechanismus bei der Nitrierung von ungesättigten Verbindungen. Nitrofluorierung von Olefinen und deren Halogensubsitutionsprodukten", Seiten 330-333**
**IZVESTIYA AKADEMII NAUK SSSR, SERIYA KHIMICHESKAYA, 1963, I.L. KNUNYANTS, L.S. GERMAN, I.N., ROZKHOV "Aliphatische Fluoronitroverbindungen. 1. Konjugierte Nitrofluorierung von Olefinen", Seiten 1946-1950**
**CHEMICAL ABSTRACTS, Band 97, Nr. 1, 5. Juli 1982, Columbus, Ohio, USA, A.G. TALYBOV et al. "Reaction**

(73) Patentinhaber: **BAYER AG, Konzernverwaltung RP Patentabteilung, D-5090 Leverkusen 1 Bayerwerk (DE)**

(72) Erfinder: ***Baasner, Bernd, Dr., Mozartstrasse* 41, D-5090 Leverkusen 1 (DE)**
Erfinder: **Hagemann, Hermann, Dr., Kandinsky-Strasse 52, D-5090 Leverkusen 1 (DE)**
Erfinder: **Klauke, Erich, Dr., Eichendorffweg 8, D-5068 Odenthal (DE)**

(56) Entgegenhaltungen: (Fortsetzung)
**of haloolefins with nitronium tetrafluoroborate", Seite 560, Zusammenfassung Nr. 5760k**

**EP 0 116 885 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein verbessertes Verfahren zur Herstellung von α-fluorierten Nitroalkanen und -cycloalkanen durch konjugierte Nitrofluorierung der entsprechenden Olefine. Die erfindungsgemäss herstellbaren α-fluorierten Nitroalkane sind zum Teil bereits bekannt und können als Zwischenprodukte zur Herstellung von bestimmten Herbiziden verwendet werden.

Fluorierte Nitroalkane können nach verschiedenen Verfahren hergestellt werden, zum Beispiel durch Umsetzung von aliphatischen, Nitrogruppen enthaltenden Carbonsäuren mit $SF_4/BF_3$ [vgl. Tetrahedron 26, S. 5737 (1970)]. Die benötigten Ausgangsstoffe müssen erst hergestellt werden, und das anschliessende Fluorierungsverfahren ist ausserordentlich aufwendig und für eine technische Anwendung völlig ungeeignet.

Weiterhin ist es durch Arbeiten russischer Autoren bekannt geworden, dass durch die Reaktion von Olefinen in wasserfreiem Fluorwasserstoff mit konzentrierter Salpetersäure aliphatische α-fluorierte Nitroverbindungen gemäss dem allgemeinen Reaktionsschema:

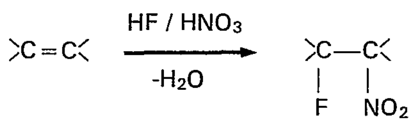


hergestellt werden können [vgl. Dokl. Akad. Nauk. SSSR 149, S. 222-5 (1963) (engl.) und Izvest. Akad. Nauk. SSSR 1963, S. 1794-7 (engl.)].

Diese Reaktion wird als konjugierte Nitrofluorierung bezeichnet und erscheint prinzipiell attraktiv für eine technische Anwendung. Es zeigt sich jedoch, dass die aus den genannten russischen Publikationen bekannten Laborvorschriften für eine direkte Übertragung in den grosstechnisch-industriellen Massstab ebenfalls völlig ungeeignet sind:

Der Fluorwasserstoff wirkt bei der vorbekannten Verfahrensweise zugleich als Fluorierungs- und als Lösungsmittel und wird in sehr grossem Überschuss eingesetzt. Da bei der Reaktion in äquimolarer Menge Wasser entsteht, fällt während der Umsetzung eine wässrige $HF/HNO_3$-Mischung an, welche auf die üblicherweise verwendeten Stahl-Rührgefässe stark korrosiv wirkt. Um diese Korrosion einzuschränkten, muss ein grosser HF-Überschuss verwendet werden; dadurch kann die schädliche Wasserkonzentration so stark herabgesetzt werden, dass sich sogar der Beständigkeitsbereich der betreffenden Reaktionsgefässwerkstoffe erreichen lässt. — Bei dem bekannten Verfahren müssen auf 1 Mol Olefin 5 bis 100 Mol Fluorwasserstoff, also wenigstens 500 Mol-% Überschuss, eingesetzt werden. Die Weiterverarbeitung der anfallenden wässrigen Säuremischung ist sehr kostenintensiv und technologisch problematisch. Eine Rückgewinnung der überschüssigen Flusssäure aus der anfallenden wässrigen Lösung in wasserfreier Form, wie sie für erneute Umsetzungen benötigt würde, ist mit vertretbarem technischen Aufwand praktisch nicht möglich.

Es wurde nun überraschend gefunden, dass die konjugierte Nitrofluorierung mit einem wesentlich geringeren molaren Überschuss an Fluorwasserstoff, nämlich mit maximal 10% Überschuss, bezogen auf eingesetztes Olefin, durchgeführt werden kann, ohne dass Ausbeuteverluste eintreten, wobei der Fluorwasserstoff in Form einer wässrigen Lösung eingesetzt wird. Gleichzeitig werden auf 1 Mol Olefin 1 bis 2 Mol Salpetersäure eingesetzt. Das neue Verfahren wird in gegen Korrosion durch wässrige Flusssäure geschützten Apparaturen durchgeführt.

Gegenstand der Erfindung ist somit ein Verfahren zur Herstellung von α-fluorierten Nitroalkanen der allgemeinen Formel

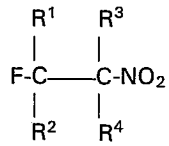 bzw. 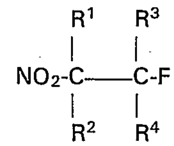 (I)

(Ia) (Ib)

in welcher

$R^1$, $R^2$, $R^3$ und $R^4$ gleich oder verschieden sind und einzeln für Wasserstoff, Fluor, Chlor, Brom, Alkyl, Halogenalkyl oder Cycloalkyl stehen oder

$R^1$ und $R^3$ die angegebene Bedeutung haben und

$R^2$ und $R^4$ gemeinsam für eine Alkylengruppe mit 3-6 C-Atomen stehen,

durch konjugierte Nitrofluorierung der entsprechenden Olefine, dadurch gekennzeichnet, dass man pro Mol Olefin der Formel

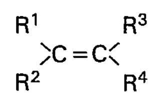 (II)

in welcher

$R^1$, $R^2$, $R^3$ und $R^4$ die oben angegebene Bedeutung haben,

1 bis maximal 1,1 Mol Fluorwasserstoff und 1 bis 2 Mol Salpetersäure einsetzt und die Umsetzung in gegen Korrosion geschützten Apparaturen durchführt, wobei der Fluorwasserstoff in Form einer wässrigen Lösung eingesetzt wird und die Konzentration des Säuregemisches in Wasser 40 bis 80% beträgt.

Ob dabei die Produkte der Formel (Ia) oder (Ib) oder — in Ausnahmefällen — Gemische von (Ia) und (Ib) gebildet werden, lässt sich anhand der bekannten Markownikow-Regel ableiten.

Durch die aufgefundene Möglichkeit, die konjugierte Nitrofluorierung mit wesentlich geringerem HF-Überschuss (d.h. maximal 10 Mol-% gegenüber 500 bis 10 000 Mol-% bei der vorbekannten Verfahrensweise) bei Erzielung gleich guter Ausbeuten an α-Fluor-nitroalkanen ausführen zu können, lassen sich erhebliche Mengen an Fluorwasserstoff einsparen. Die Reaktion kann damit viel kostengünstiger und sicherer durchgeführt werden und kommt jetzt auch für eine Anwendung im grosstechnischen Massstab in Betracht.

Die als Ausgangsstoffe einzusetzenden Olefine

sind durch die Formel (II) allgemein definiert. In dieser Formel stehen $R^1$, $R^2$, $R^3$ und $R^4$, die gleich oder verschieden sein können, vorzugsweise für Wasserstoff, Fluor, Chlor, Brom, Alkyl mit 1-4 C-Atomen, Halogenalkyl mit 1-4 C-Atomen (und bevorzugt mit Fluor und Chlor als Halogenatom) oder für Cycloalkyl mit 3-8 C-Atomen, oder es stehen $R^1$ und $R^3$ für die zuvor angegebenen Reste, und $R^2$ und $R^4$ stehen gemeinsam für eine Alkylengruppe mit 3-4 C-Atomen.

Die erfindungsgemäss verwendbaren Olefine sind bereits bekannt oder können nach allgemein bekannten Methoden hergestellt werden. Es können beispielsweise die im folgenden genannten Olefine als Ausgangsstoffe eingesetzt werden:

Chlorethen, 1,1-Dichlorethen, 1,1-Difluorethen, Fluorethen, Tetrafluorethen, Trifluorchlorethen, 1,2-Dichlor-1,2-difluorethen, Trifluorethen, 1--Chlor-1,2-difluorethen, 1,1-Dichlor-2-fluorethen, Ethen, 1-Chlor-1-fluorethen, 1,1-Dichlor-2,2-difluorethen, Trichlorethen, 1,2-Dichlor-1-fluorethen, Propen, 1,1-Difluorpropen, 1,1-Dichlorpropen, 1--Chlor-1-fluorpropen, 2-Fluorpropen, 2-Chlorpropen, 1,1-Dichlor-3,3-dimethylpropen-1, Hexafluorpropen, 1,1,3,3,3-Pentafluorpropen, 1,1-Dichlor-2,3,3,3-tetrafluorpropen, 1,1,2-Trichlor--3,3,3-trifluorpropen, 1,1,2,3-Tetrachlor-3,3-difluorpropen, 3-Chlorpropen, 2,3-Dichlorpropen, 3,3,3-Trifluorpropen, Bromethen, 1,1-Difluorbuten, 1-Chlor-1-fluorbuten, 1,1-Dichlorbuten, Cyclohexen, 2-(Fluormethyl)-3-fluorpropen-1.

Das erfindungsgemässe Verfahren wird in gegen Korrosion geschützten Apparaturen, die beispielsweise aus Teflon, Polyethylen oder anderen, gegen wässrige Flusssäure inerten Materialien bestehen, oder in mit diesen Materialien ausgekleideten Stahl-Rührgefässen, in denen drucklos oder bei erhöhtem Druck gearbeitet werden kann, durchgeführt. Die Reihenfolge, in der die Reaktionskomponenten miteinander gemischt werden, ist dabei im Prinzip beliebig.

Es ist jedoch besonders zweckmässig, zur Durchführung des Verfahrens in einem entsprechenden, gegen wässrige Flusssäure korrosionsbeständigen Rührgefäss oder Rührautoklaven eine Mischung von wässriger Flusssäure und Salpetersäure vorzulegen und das Olefin dann zuzufügen.

Der Überschuss an Fluorwasserstoff kann zwar im Verhältnis zum eingesetzten Olefin beliebig gross sein, jedoch ist im Sinne der Erfindung ein Molverhältnis von 1 bis 1,1 Mol Fluorwasserstoff pro Mol Olefin (II) voll ausreichend.

Auf 1 Mol Olefin (II) werden ausserdem 1 bis 2 Mol Salpetersäure eingesetzt. Bevorzugt ist ein Verhältnis von 1 bis 1,2 Mol Salpetersäure pro Mol Olefin, besonders bevorzugt von 1 Mol Salpetersäure pro Mol Olefin.

Die Konzentration des Säuregemisches in Wasser beträgt 40 bis 80%, besonders bevorzugt 50 bis 70%. Zwar findet die Reaktion auch bei hohen Verdünnungen statt, aufgrund schlechter Löslichkeit der umzusetzenden Olefine im Säuregemisch verlängert sich die Reaktionszeit jedoch unverhältnismässig.

Nach dem Vorlegen des Säuregemisches wird bei Temperaturen von —80° bis +120°C, bevorzugt von —60° bis +80°C das umzusetzende Olefin in das Reaktionsgefäss gebracht, indem es eingeleitet, einkondensiert, zugetropft oder in das verschlossene Reaktionsgefäss eingedrückt wird. Die Reaktionstemperaturen selbst liegen ebenfalls zwischen —80° und +120°C, bevorzugt zwischen —20° und +80°C. Die Reaktion erfolgt bei Normaldruck oder dem Eigendruck, der sich im Verlauf der Reaktion im verschlossenen Reaktionsgefäss aufbaut. Es kann aber auch unter höheren Drücken, etwa bis 50 bar, gearbeitet werden, wobei dieser Druck durch Aufpressen eines Inertgases wie Stickstoff erreicht wird. Die Reaktionsdauer beträgt 0,5 bis 48 Stunden, in der Regel reicht eine Reaktionszeit von 2 bis 16 Stunden aus.

Die Aufarbeitung und Isolierung der Reaktionsprodukte erfolgt nach üblichen Methoden.

Die erfindungsgemäss herstellbaren α-Fluor-nitroalkane (I) können z.B. als Zwischenprodukte zur Herstellung von fluorhaltigen herbiziden Wirkstoffen, z.B. aus der Reihe der sym-Triazine verwendet werden. Die Nitrogruppe in den Verbindungen (I) lässt sich durch katalytische Hydrierung glatt zur Aminogruppe reduzieren, wodurch man in hohen Ausbeuten die entsprechenden α-fluorierten Alkyl- bzw. Cycloalkylamine erhält. Diese lassen sich in bekannter Weise mit Cyanurchlorid oder Cyanurfluorid zu bekannten sym-Triazinen umsetzen, welche durch entsprechende Fluoralkylaminogruppen substituiert sind und bekanntermassen starke herbizide Eigenschaften aufweisen (vgl. z.B. DE-OS 3 127 861, DE-OS 3 218 201, DE-OS 3 218 966).

So erhält man beispielsweise ausgehend von 1-Methyl-2,2,2-trifluornitroethan auf folgendem Wege den herbiziden Wirkstoff 2-Chlor-4-ethylenamino-6-(1-methyl-2,2,2-trifluormethyl-amino)-s--triazin:

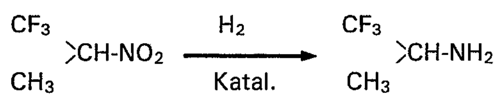


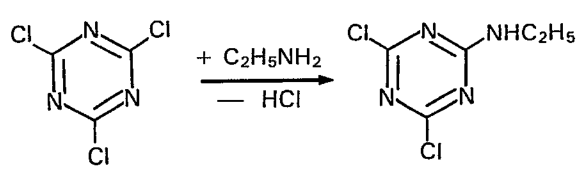


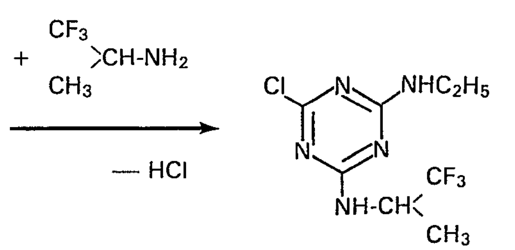

Die nachfolgenden Beispiele dienen zur weiteren Erläuterung der Erfindung.

*Beispiele*

*Beispiel 1*

$CF_3$-$CH_2$-$NO_2$

a) *gemäss der Erfindung:*

In einem mit Polyethylen ausgekleideten Rührgefäss wurden 220 g (11 Mol) Fluorwasserstoff, 780 g (43,3 Mol) Wasser und 630 g (10 Mol) Salpetersäure (d = 1,51) vorgelegt, und unter Kühlung wurden bei 0° bis +10°C 640 g (10 Mol) 1,1-Difluorethen eingeleitet. Man liess das Reaktionsgemisch auf Raumtemperatur kommen und 8 Stunden nachrühren. Anschliessend wurde das Reaktionsgemisch mit 1 Liter Wasser verdünnt, die organische Phase abgetrennt und die wässrige dreimal mit je 200 ml Dichlormethan extrahiert. Die vereinigten organischen Phasen wurden mit Natriumhydrogencarbonat-Lösung und Wasser neutral gewaschen und über Magnesiumsulfat getrocknet. Nach Abdestillieren des Lösungsmittels erhielt man 850 g (≙ 66% d.Th.) 2,2,2-Trifluornitroethan vom Siedepunkt 91-92°C; $n_D^{20}$: 1.3290.

b) *Vergleichsversuch*

[gemäss Izvest. Akad. Nauk. SSSR 1963, S. 1794-7 (engl.)]:

In einem Stahlrührautoklaven wurden 1400 g (70 Mol) Fluorwasserstoff (wasserfrei) und 630 g (10 Mol) Salpetersäure (d = 1,51) vorgelegt und unter Solekühlung bei —30° bis —10°C 640 g (10 Mol) 1,1-Difluorethen eingeleitet. Man liess in etwa 4 Stunden auf Raumtemperatur kommen und goss das Reaktionsgemisch dann auf 1,5 kg Eis, trennte die organische Phase ab und extrahierte die wässrige dreimal mit je 200 ml Dichlormethan. Die vereinigten organischen Phasen wurden mit Natriumhydrogencarbonat-Lösung und Wasser neutral gewaschen, anschliessend über Magnesiumsulfat getrocknet. Nach Abdestillieren des Lösungsmittels erhielt man 790 g (≙ 61% d.Th.) 2,2,2-Trifluornitroethan vom Siedepunkt 91-92°C; $n_D^{20}$: 1.3285.

Analog zu Beispiel 1a) wurden die in der folgenden Tabelle 1 aufgeführten Verbindungen hergestellt:

TABELLE 1

| Beispiel Nr. | Olefin (II) | Produkt (I) | Siedepunkt [°C] | $n_D^{20}$ | Ausbeute (%) |
|---|---|---|---|---|---|
| 2 | $CHCl=CH_2$ | $CFClH$-$CH_2$-$NO_2$ | 55-7/40 mbar | 1.4235 | 65 |
| 3 | $CHF=CH_2$ | $CF_2H$-$CH_2$-$NO_2$ | 25/20 mbar | 1.3648 | 69 |
| 4 | $CCl_2=CH_2$ | $CFCl_2$-$CH_2$-$NO_2$ | 57/30 mbar | 1.4370 | 82 |
| 5 | $CBrH=CH_2$ | $CFBrH$-$CH_2$-$NO_2$ | 65-6/30 mbar | 1.4658 | 56 |
| 6 | $CCl_2=CH$-$CH_3$ | $CFCl_2$-CH-$CH_3$<br>\|<br>$NO_2$ | 55/20 mbar | 1.4390 | 69 |
| 7 | $CFCl=CH$-$CH_3$ | $CF_2Cl$-CH-$CH_3$<br>\|<br>$NO_2$ | 34-5/20 mbar | 1.3870 | 75 |
| 8 | $CF_2=CH$-$CH_3$ | $CF_3$-CH-$CH_3$<br>\|<br>$NO_2$ | 99-100/1 bar | 1.3386 | 82 |
| 9 | $CH_3$-$CF=CH_2$ | $CH_3$-$CF_2$-$CH_2$-$NO_2$ | 40-2/25 mbar | 1.3704 | 76 |
| 10 | $CFCl=CH_2$ | $CF_2Cl$-$CH_2$-$NO_2$ | 55-6/30 mbar | 1.3828 | 74 |

Die α-Fluornitroalkane der Beispiele 3, 6, 7, 9 und 10 sind neue Verbindungen.

**Patentansprüche**

1. Verfahren zur Herstellung von α-fluorierten Nitroalkanen der allgemeinen Formel

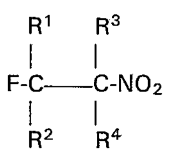 bzw. 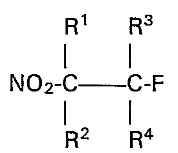 (I)

(Ia) (Ib)

in welcher

$R^1$, $R^2$, $R^3$ und $R^4$ gleich oder verschieden sind und einzeln für Wasserstoff, Fluor, Chlor, Brom, Alkyl, Halogenalkyl oder Cycloalkyl stehen oder

$R^1$ und $R^3$ die angegebene Bedeutung haben und $R^2$ und $R^4$ gemeinsam für eine Alkylengruppe mit 3-6 C-Atomen stehen,

durch konjugierte Nitrofluorierung der entsprechenden Olefine, dadurch gekennzeichnet, dass man pro Mol Olefin der Formel

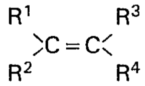

(II)

in welcher

$R^1$, $R^2$, $R^3$ und $R^4$ die oben angegebene Bedeutung haben,

1 bis maximal 1,1 Mol Fluorwasserstoff und 1 bis 2 Mol Salpetersäure einsetzt und die Umsetzung in gegen Korrosion geschützten Apparaturen durchführt, wobei der Fluorwasserstoff in Form einer wässrigen Lösung eingesetzt wird und die Konzentration des Säuregemisches im Wasser 40 bis 80% beträgt.

2. Verfahren nach Anspruch 1, dadurch gekennzeichnet, dass man bei Temperaturen zwischen —80 und +120°C arbeitet.

3. Verfahren nach Anspruch 2, dadurch gekennzeichnet, dass man bei Temperaturen zwischen —20 und +80°C arbeitet.

4. Verfahren nach Anspruch 1, dadurch gekennzeichnet, dass man im Druckbereich von 1-50 bar arbeitet.

5. Verfahren nach Anspruch 1, dadurch gekennzeichnet, dass man auf 1 Mol Olefin (II) 1-1,2 Mol Salpetersäure einsetzt.

**Claims**

1. Process for the preparation of α-fluorinated nitroalkanes of the general formula

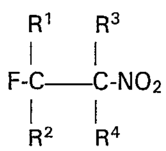

or

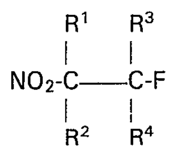

(I)

(Ia) (Ib)

in which

$R^1$, $R^2$, $R^3$ and $R^4$ are identical or different and individually represent hydrogen, fluorine, chlorine, bromine, alkyl, halogenoalkyl or cycloalkyl, or

$R^1$ and $R^3$ have the meaning indicated and

$R^2$ and $R^4$ together represent an aklylene group having 3-6 carbon atoms,

by conjugated nitrofluorination of the corresponding olefines, characterised in that 1 to a maximum of 1.1 mol of hydrogen fluoride and 1 to 2 mol of nitric acid are employed per mol of olefine of the formula

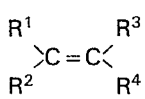

(II)

in which

$R^1$, $R^2$, $R^3$ and $R^4$ have the meaning indicated above,

and the reaction is carried out in equipment protected against corrosion, the hydrogen fluoride being empoyed in the form of an aqueou solution and the concentration of the acid mixture being 40 to 80%.

2. Process according to Claim 1, characterised in that it is carried out at temperatures between —80 and +120°C.

3. Process according to Claim 2, characterised in that it is carried out at temperatures between —20 and +80°C.

4. Process according to Claim 1, characterised in that it is carried out in the pressure range 1-50 bar.

5. Process according to Claim 1, characterised in that 1-1.2 mol of nitric acid is employed to 1 mol of olefine (II).

**Revendications**

1. Procédé pour préparer des nitroalcane α-fluorés, de formule générale:

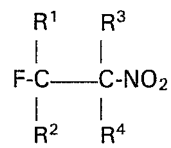

ou

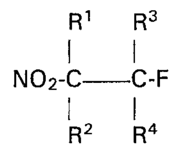

(I)

(Ia) (Ib)

dans lesquels

$R^1$, $R^2$, $R^3$ et $R^4$ sont identiques ou diffétents et représentent chacun un atome d'hydrogène, de fluor, de chlore, de brome, un groupe alkyle, halogénoalkyle ou cycloalkyle, ou bien

$R^1$ et $R^3$ ont le sens indiqué et,

$R^2$ et $R^4$ forment ensemble un groupe alkylène ayant 3 à 6 atomes de carbone,

par nitrofluoration conjugée des oléfines correspondantes, procédé caractérisé en ce qu'on utilise, par mole de l'oléfine de formule:

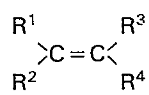

(II)

dans laquelle

$R^1$, $R^2$, $R^3$ et $R^4$ ont le sens indiqué ci-dessus,

1 à 1,1 mole au maximum de fluorure d'hydrogène et 1 à 2 moles d'acides nitrique et l'on conduit la réaction dans les appareillages protégés contre la corrosion, le fluorure d'hydrogène étant utilisé sous forme d'une solution aqueuse, et la concentration du mélange des acides dans l'eau étant de 40 à 80%.

2. Procédé selon la revendication 1, caractérisé en ce qu'on travaille à des températures comprises entre —80 et +120°C.

3. Procédé selon la revendication 2, caractérisé en ce qu'on travaille à des températures comprises entre —20 et +80°C.

4. Procédé selon la revendication 1, caractérisé en ce qu'on travaille dans un intervalle de la pression allant de 1 à 50 bars.

5. Procédé selon la revendication 1, caractérisé en ce qu'on utilise, pour 1 mole de l'oléfine (II), 1 à 1,2 mole d'acide nitrique.